(19) **Europäisches Patentamt / European Patent Office / Office européen des brevets**

(11) **EP 3 455 184 B1**

(12) # EUROPEAN PATENT SPECIFICATION

(45) Date of publication and mention
of the grant of the patent:
**08.12.2021 Bulletin 2021/49**

(21) Application number: **17722474.8**

(22) Date of filing: **11.05.2017**

(51) Int Cl.:
*C03C 25/10* (2018.01)        *C03C 25/26* (2018.01)
*C09J 189/00* (2006.01)       *D04H 1/64* (2012.01)
*D04H 3/004* (2012.01)        *D04H 3/002* (2012.01)
*E04B 1/74* (2006.01)

(86) International application number:
**PCT/EP2017/061416**

(87) International publication number:
**WO 2017/194722 (16.11.2017 Gazette 2017/46)**

(54) **MINERAL WOOL PRODUCT**

PRODUKT AUS MINERALWOOLE

PRODUIT DE LAINE MINERALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2016 EP 16169638
13.05.2016 EP 16169635
13.05.2016 EP 16169641**

(43) Date of publication of application:
**20.03.2019 Bulletin 2019/12**

(73) Proprietor: **Rockwool International A/S
2640 Hedehusene (DK)**

(72) Inventor: **HJELMGAARD, Thomas
3480 Fredensborg (DK)**

(74) Representative: **Letzelter, Felix Phillip
Meissner Bolte Patentanwälte
Rechtsanwälte Partnerschaft mbB
Widenmayerstraße 47
80538 München (DE)**

(56) References cited:
**EP-A1- 2 738 232        WO-A1-97/19141
WO-A1-2010/132641        CN-A- 101 302 410
DE-A1- 4 130 077**

- **PENA C ET AL: "Enhancing water repellence and
mechanical properties of gelatin films by tannin
addition", BIORESOURCE TECHNOLOGY,
ELSEVIER BV, GB, vol. 101, no. 17, 1 September
2010 (2010-09-01), pages 6836-6842,
XP027052600, ISSN: 0960-8524 [retrieved on
2010-05-15]**

**Description**

**Field of the Invention**

The present invention relates to a mineral wool product made with a binder, and a method of producing a mineral wool product with the binder.

**Background of the Invention**

Mineral fibre products generally comprise man-made vitreous fibres (MMVF) such as, e.g., glass fibres, ceramic fibres, basalt fibres, slag wool, mineral wool and stone wool (rock wool), which are bonded together by a cured thermoset polymeric binder material. For use as thermal or acoustical insulation products, bonded mineral fibre mats are generally produced by converting a melt made of suitable raw materials to fibres in conventional manner, for instance by a spinning cup process or by a cascade rotor process. The fibres are blown into a forming chamber and, while airborne and while still hot, are sprayed with a binder solution and randomly deposited as a mat or web onto a travelling conveyor. The fibre mat is then transferred to a curing oven where heated air is blown through the mat to cure the binder and rigidly bond the mineral fibres together.

In the past, the binder resins of choice have been phenol-formaldehyde resins which can be economically produced and can be extended with urea prior to use as a binder. However, the existing and proposed legislation directed to the lowering or elimination of formaldehyde emissions have led to the development of formaldehyde-free binders such as, for instance, the binder compositions based on poly-carboxy polymers and polyols or polyamines, such as disclosed in EP-A-583086, EP-A-990727, EP-A-1741726, US-A-5,318,990 and US-A-2007/0173588.

Another group of non-phenol-formaldehyde binders are the addition/-elimination reaction products of aliphatic and/or aromatic anhydrides with alkanolamines, e.g., as disclosed in WO 99/36368, WO 01/05725, WO 01/96460, WO 02/06178, WO 2004/007615 and WO 2006/061249. These binder compositions are water soluble and exhibit excellent binding properties in terms of curing speed and curing density. WO 2008/023032 discloses urea-modified binders of that type which provide mineral wool products having reduced moisture take-up.

Since some of the starting materials used in the production of these binders are rather expensive chemicals, there is an ongoing need to provide formaldehyde-free binders which are economically produced.

A further effect in connection with previously known aqueous binder compositions from mineral fibres is that at least the majority of the starting materials used for the productions of these binders stem from fossil fuels. There is an ongoing trend of consumers to prefer products that are fully or at least partly produced from renewable materials and there is therefore a need to provide binders for mineral wool which are at least partly produced from renewable materials.

A further effect in connection with previously known aqueous binder compositions for mineral fibres is that they involve components which are corrosive and/or harmful. This requires protective measures for the machinery involved in the production of mineral wool products to prevent corrosion and also requires safety measures for the persons handling this machinery. This leads to increased costs and health issues and there is therefore a need to provide binder compositions for mineral fibres with a reduced content of corrosive and/or harmful materials.

A yet further effect in connection with previously known aqueous binder compositions from mineral fibres is that these binders are conventionally associated with extensive curing equipment for curing the binder. The curing equipment is conventionally an oven operating at temperatures far above 100 °C such as around 200 °C. The oven is several meters long to accommodate the web that is continuously fed into the oven and to ensure that the web is fully cured when leaving the oven. Such oven equipment is associated with extensive energy consumption.

The reference C. Peña, K. de la Caba, A. Eceiza, R. Ruseckaite, I. Mondragon in Biores. Technol. 2010, 101, 6836-6842 is concerned with the replacement of non-biodegradable plastic films by renewable raw materials from plants and wastes of meat industry. In this connection, this reference describes the use of hydrolysable chestnut-tree tannin for modification of a gelatin in order to form films. The reference does not describe binders, in particular not binders for mineral wool.

WO 2010/132641 A1 relates to an aqueous binder composition for use in the formation of fiberglass insulation and non-woven mats comprising at least one protein-based biomass, and at least one pH adjusting agent, wherein the protein based biomass can be selected from different sources, one member of the list mentioned being soy.

DE 4130077 A1 is directed to a sizing composition for glass fibres which is supposed to improve the durability of said fibres to aggressive media. According to the examples of this document, a sizing composition comprising gelatine is applied on the glass fibres and then dried at 130 °C.

CN 101302410 A is directed to a method for preparing an adhesive from proteins grafted with vinyl monomers. The adhesive can be used for adhesion of wood.

EP 2738232 A1 is directed to an adhesive composition comprising a hydrolysable tannin and at least one biopolymer used for wood. The adhesive composition may comprise tannic acid and added gelatin and/or a polylysine solution.

WO 97/19141 is directed to a method of manufacturing an adhesive comprising crosslinking first and second molecules each of which has at least one group selected from the group of hydroxyl aromatic, dihydroxy aromatic, hydroxy phenole and amino, the first molecule being linked to the second molecule via at least one quinone.

**Summary of the Invention**

Accordingly, it was an object of the present invention to provide a binder composition which is particularly suitable for bonding mineral fibres, uses renewable materials as starting materials, reduces or eliminates corrosive and/or harmful materials.

Further, it was an object of the present invention to provide a binder composition which does not require high temperature for curing and therefore eliminates need of high temperature to be applied in the production of a product bonded with the binder composition.

A further object of the present invention was to provide a mineral wool product bonded with such a binder composition.

A further object of the present invention was to provide a method of making such mineral wool product.

A further object of the present invention was to provide the use of such a binder composition for the preparation of the mineral wool product.

In accordance with a first aspect of the present invention, there is provided a mineral wool product comprising mineral fibres bound by a binder resulting from the curing of a formaldehyde-free binder composition for mineral fibres comprising:

- at least one phenol containing compound,
- at least one protein,

    wherein the content of phenol containing compound is 1-70 wt.%, such as 2 to 60 wt.%, such as 3 to 50 wt.%, such as 4 to 40 wt.%, such as 5 to 35 wt.%, based on dry protein basis,
    wherein the at least one protein is selected from the group consisting of proteins from animal sources, including collagen, gelatine, hydrolysed gelatine, and protein from milk (casein, whey), eggs, polyphenolic proteins such as mussel foot protein.

In accordance with a second aspect of the present invention, there is provided a method of producing such a mineral wool product which comprises the steps of contacting mineral fibres with such a binder composition, wherein the curing is carried out at temperatures from 5 to 95 °C, such as 10 to 60 °C, such as 20 to 40 °C.

The present inventors have surprisingly found that it is possible to obtain a mineral wool product comprising mineral fibres bound by a binder resulting from the curing of a binder composition, whereby the binder composition can be produced from renewable materials to a large degree, does not contain, or contains only to a minor degree, any corrosive and/or harmful agents and the production of the mineral wool product does not lead to pollution such as VOC's (Volatile Organic Compounds) during the preparation.

**Description of the Preferred Embodiments**

The binder composition used in the present invention comprises:

- at least one phenol containing compound,
- at least one protein.

The binders used in the present invention are formaldehyde free.

For the purpose of the present application, the term "formaldehyde free" is defined to characterize a mineral wool product where the emission is below 5 $\mu$g/m$^2$/h of formaldehyde from the mineral wool product, preferably below 3 $\mu$g/m$^2$/h. Preferably, the test is carried out in accordance with ISO 16000 for testing aldehyde emissions.

A surprising advantage of embodiments of mineral wool products according to the present invention is that they show self-healing properties. After being exposed to very harsh conditions when mineral wool products loose a part of their strength, the mineral wool products according to the present invention can regain a part of the original strength. This is in contrast to conventional mineral wool products for which the loss of strength after being exposed to harsh environmental conditions is irreversible. While not wanting to be bound to any particular theory, the present inventors believe that this surprising property in mineral wool products according to the present invention is due to the complex nature of the bonds formed in the network of the protein crosslinked by the phenol containing compound which also includes quaternary structures and hydrogen bonds and allows bonds in the network to be established after returning to normal environmental conditions. For an insulation product, which when e.g. used as a roof insulation can be exposed to very high temperatures in the summer, this is an important advantage for the long term stability of the product.

Phenol containing compound component of the binder

The binder composition used in the present invention comprises a phenol containing compound component of the binder, in particular one or more phenolic compound.

Phenolic compounds, or phenolics, are compounds that have one or more hydroxyl group attached directly to an aromatic ring. Polyphenols (or polyhydroxyphenols) are compounds that have more than one phenolic hydroxyl group attached to one or more aromatic rings. Phenolic compounds are characteristic of plants and as a group they are usually found as esters or glycosides rather than as free compounds.

The term phenolics covers a very large and diverse group of chemical compounds. Preferably, the phenol containing compound is a compound according to the scheme based on the number of carbons in the molecule as detailed in by W. Vermerris, R. Nicholson, in Phenolic Compound Biochemistry, Springer Netherlands, 2008.

Preferably, the phenol containing compound is in form of one or more components selected from the group consisting of a compound with a $C_6$ structure such as simple phenolics, such as resorcinol, phloroglucinol, such as a compound with a $C_6$-$C_1$ structure such as hydroxybenzoic acids, such as p-hydroxybenzoic acid, gallic acid, protocathechuic acid, salicylic acid, vanillic acid, such as hydroxybenzoic aldehydes, such as vanillin, such as a compound with a $C_6$-$C_2$ structure such as hydroxyacetophenones, such as 2-hydroxyacetophenone, such as hydroxyphenylacetic acids, such as 2-hydroxyphenyl acetic acid, such as a compound with a $C_6$-$C_3$ structure such as cinnamic acids, such as p-coumaric acid, caffeic acid, ferulic acid, 5-hydroxyferulic acid, sinapic acid, such as cinnamic acid esters, such as chlorogenic acid, sinapoyl malate, sinapoyl choline, such as cinnamyl aldehydes, such as cinnamyl alcohols, such as coumarins, such as umbelliferone, 4-methyl umbelliferone, such as isocoumarins, such as bergenin, such as chromones, such as a compound with a $C_{15}$ structure such as flavonoids, such as flavanone, isoflavones, iso-flavanones, neoflavanoids, such as chalcones, such as butein, such as dihydrochalcones, such as phloridzin, such as aurones, such as flavanones, such as naringenin, such as flavanonols, such as taxifolin, such as flavans, such as leucoanthocyanidins, such as leucocyanidin, leucodelphinidin, such as flavan-3-ols, such as catechin, gallocatechin, such as flavones, such as kaemferol, quercetin, myricetin, such as anthocyanidins, such as pelargonidin, cyanidin, peonidin, delphinidin, petunidin, malvidin, such as deoxyanthocyanidines, such as apigeninidin, luteolinidin, 7-meth-oxyapigeninidin, 5-methoxy-luteolinidin, such as anthocyanins, such as petanin, such as a compound with a $C_{30}$ structure such as biflavonyls, such as ginkgetin, such as a compound with a $C_6$-$C_1$-$C_6$ structure such as benzophenones, such as xanthones, such as a compound with a $C_6$-$C_2$-$C_6$ structure such as stilbenes, such as resveratrol, pinosylvin, such as a compound with a $C_6$ / $C_{10}$ / $C_{14}$ structure such as benzoquinones, such as naphthaquinones, such as juglone, such as anthraquinones, such as emodin, such as a compound with a $C_{18}$ structure such as betacyanins, such as betanidin, such as polyphenols and/or polyhydroxyphenols, such as lignans, neolignans (dimers or oligomers from coupling of monolignols such as p-coumaryl alcohol, coniferyl alcohol and sinapyl alcohol), such as pinoresinol, sesamin, plicatic acid, such as lignins (synthesized primarily from the monolignol precursors p-coumaryl alcohol, coniferyl alcohol and sinapyl alcohol), such as tannins, such as condensed tannins (proanthocyanidins), such as procyanidin $B_2$, such as hydrolysable tannins, such as gallotannins, such as ellagitannins, such as complex tannins, such as acutissimin A, such as tannic acid, such as phlobabenes.

In a preferred embodiment, the phenol containing compound is selected from the group consisting of simple phenolics, phenol containing compounds with a more complex structure than a $C_6$ structure, such as oligomers of simple phenolics, polyphenols, and/or polyhydroxyphenols.

The phenol containing compounds can also be synthetic or semisynthetic molecules or constructs that contain phenols, and/or polyphenols . An example for such a construct is a protein, peptide, peptoids (such as linear and/or cyclic oligomers and/or polymers of N-substituted glycines, N-substituted β-alanines), or arylopeptoids (such as linear and/or cyclic oligomers and/or polymers of N-substituted aminomethyl benzamides) modified with phenol containing side chains. A dendrimer decorated with phenol side chains is another example.

Tannins comprise a group of compounds with a wide diversity in structure that share their ability to bind and precipitate proteins. Tannins are abundant in many different plant species, in particular oak, chestnut, staghorn sumac and fringe cups. Tannins can be present in the leaves, bark and fruits. Tannins can be classified into three groups: condensed tannins, hydrolysable tannins and complex tannins. Condensed tannins, or proanthocyanidins, are oligomeric or polymeric flavonoids consisting of flavan-3-ol (catechin) units. Gallotannins are hydrolysable tannins with a polyol core substituted with 10-12 gallic acid residues. The most commonly found polyol in gallotannins is D-glucose although some gallotannins contain catechin and triterpenoid units as the core polyol. Ellagitanins are hydrolysable tannins that differ from gallotannins in that they contain additional C-C bonds between adjacent galloyl moieties. Complex tannins are defined as tannins in which a catechin unit is bound glycosidically to either a gallotannin or an ellagitannin unit.

The inventors have surprisingly found that a wide range of such phenol containing compounds can be used to crosslink proteins which allows a binder composition to be formed. Often, these phenol containing compound components are obtained from vegetable tissues and are therefore a renewable material. In some embodiments, the compounds are also non-toxic and non-corrosive. As a further advantage, these compounds are antimicrobial and therefore impart their antimicrobial properties to the mineral wool product bound by such a binder.

In a preferred embodiment, the phenol containing compound is selected from one or more components from the group consisting of tannic acid, ellagitannins and gallotannins, tannin originating from one or more of oak, chestnut, staghorn sumac and fringe cups.

Protein component of the binder

The protein component of the binder is in form of one or more proteins selected from the group consisting of proteins from animal sources, including collagen, gelatine, hydrolysed gelatine, and protein from milk (casein, whey), eggs; polyphenolic proteins such as mussel foot protein.

Collagen is a very abundant material in living tissue: It is the main component in connective tissue and constitutes 25-35% of the total protein content in mammals. Gelatin is derived from chemical degradation of collagen. Gelatin is water soluble and has a molecular weight of 30.000 to 300.000 g/mol dependent on the grade of hydrolysis. Gelatin is a widely used food product and it is therefore generally accepted that this compound is totally non-toxic and therefore no precautions are to be taken when handling gelatin.

The gelatin can also be further hydrolysed to smaller fragments of down to 3000 g/mol.

In a preferred embodiment, the protein component is gelatin, whereby the gelatin is preferably originating from one or more sources from the group consisting of mammal, bird species, such as from cow, pig, horse, fowl, and/or from scales, skin of fish.

In a particular preferred embodiment, the phenol containing compound component is a tannin and/or tannic acid, and the protein component is gelatin, in particular gelatin from porcine skin, in particular of medium gel strength, or low gel strength.

Reaction of the binder components

Without wanting to be bound to any particular theory, the present inventors believe that the reaction between the phenol containing compound and the protein at least partly relies on a oxidation of phenols to quinones followed by nucleophilic attack of amine and/or thiol groups from the protein which leads to a crosslinking of the proteins by the phenol containing compounds.

The content of the phenol containing compound in the binder composition used in the present invention is from 1 to 70 wt.%, such as 2 to 60 wt.%, such as 3 to 50 wt.%, such as 4 to 40 wt.%, such as 5 to 35 wt.%, based on dry protein basis.

In an preferred embodiment, the mass ratio of (lysine + cystein) in the protein to (phenol) in the phenol containing compound is 1:5.78 - 1:0.08, such as 1:2.89 - 1:0.09, such as 1:1.93 - 1:0.12, such as 1:1.45 - 1:0.15, such as 1:1.16 - 1:0.17.

The term mass ratio of (lysine + cystein) in the protein to (phenol) in the phenol containing compound is to be understood to mean the ratio of the combined mass of the lysine + cystein residues in the proteine component to the combined mass of the phenol residues in the phenole containing compound.

The present inventors have found that the curing of the binder is strongly accelerated under alkaline conditions. Therefore, in one embodiment, the binder composition for mineral fibres comprises a pH-adjuster, preferably in form of a base, such as organic base, such as amine or salts thereof, inorganic bases, such as metal hydroxide, such as KOH or NaOH, ammonia or salts thereof.

In a particular preferred embodiment, the pH adjuster is an alkaline metal hydroxide, in particular NaOH.

In a preferred embodiment, the binder composition according to the present invention has a pH of 7 to 10, such as 7.5 to 9.5, such as 8 to 9.

In one embodiment, the protein comprises polyphenolic proteins.

These proteins contain a high level of a post-translationally modified—oxidized— form of tyrosine, L-3,4-dihydroxyphenylalanine (levodopa, L-DOPA). See also J. J. Wilker Nature Chem. Biol. 2011, 7, 579-580 for a reference to these proteins.

Additives

In a preferred embodiment, the binder composition used in the present invention contains additives.

Other additives may be components such as one or more reactive or nonreactive silicones and may be added to the binder. Preferably, the one or more reactive or nonreactive silicone is selected from the group consisting of silicone constituted of a main chain composed of organosiloxane residues, especially diphenylsiloxane residues, alkylsiloxane residues, preferably dimethylsiloxane residues, bearing at least one hydroxyl, acyl, carboxyl or anhydride, amine, epoxy or vinyl functional group capable of reacting with at least one of the constituents of the binder composition and is preferably present in an amount of 0.1-15 weight-%, preferably from 0.1-10 weight-%, more preferably 0.3-8 weight-%, based on the total binder mass.

In one embodiment, an emulsified hydrocarbon oil may be added to the binder.

As already described above, many phenol containing compounds, in particular polyphenols, have antimicrobial properties and therefore impart antimicrobial characteristic to the binder. Nevertheless, in one embodiment, an anti-fouling agent may be added to the binder compositions.

In one embodiment, an anti-swelling agent may be added to the binder, such as tannic acid and/or tannins.

In one embodiment, the binder composition used in the present invention contains additives in form of amine linkers and/or thiol/thiolate linkers. These additives in form of amine linkers and/or thiol/thiolate linkers are particular useful when the crosslinking reaction of the binder proceeds via the quinone-amine and/or quinonethiol pathway.

In one embodiment, the binder compositions used in the present invention comprise an additive containing metal ions, such as iron ions.

Polyphenolic proteins such as the mussel adhesive protein discussed above relies on 3,4-dihydroxyphenyl moieties to enhance the surface adhesion. This is achieved in combination with the secretion of selected types of cations such as iron ions. In one embodiment, the binder could be said to mimic the polyphenolic protein and therefore the addition of various cations could improve the binder characteristics. Such advantageous ions can also be released from the mineral fibre surface when they come into contact with the aqueous binder.

In one embodiment, the mineral wool product comprises rock wool. Without being bound by theory, it is believed that leaching of certain ions from the vitreous fibres may assist the binding strength. The mechanism may be analogue to the mechanism for which mussel adhesive protein obtains a surface adhesion. This is achieved in combination with the secretion of selected types of cations such as iron ions.

In one embodiment, the binder compositions used in the present invention contain further additives in form of additives selected from the group consisting of PEG-type reagents, silanes, and hydroxylapatites.

Oxidising agents as additives can serve to increase the oxidising rate of the phenolics. One example is the enzyme tyrosinase which oxidizes phenols to hydroxy-phenols/quinones and therefore accelerates the binder forming reaction.

In another embodiment, the oxidising agent is oxygen, which is supplied to the binder.

In one embodiment, the curing is performed in oxygen-enriched surroundings.

A mineral wool product comprising mineral wool fibres bound by a binder

The present invention is directed to a mineral wool product bound by a binder resulting from the curing of the binder composition described.

In a preferred embodiment, the density of the mineral wool product is in the range of 10-1200 kg/m$^3$, such as 30-800 kg/m$^3$, such as 40-600 kg/m$^3$, such as 50-250 kg/m$^3$, such as 60-200 kg/m$^3$.

In a preferred embodiment, the mineral wool product according to the present invention is an insulation product, in particular having a density of 10 to 200 kg/m$^3$.

In an alternative embodiment, the mineral wool product according to the present invention is a facade panel, in particular having a density of approximately 1200 kg/m$^3$.

In a preferred embodiment, the mineral wool product according to the present invention is an insulation product.

In a preferred embodiment, the loss on ignition (LOI) of the mineral wool product according to the present invention is within the range of 0.1 to 25.0 %, such as 0.3 to 18.0 %, such as 0.5 to 12.0 %, such as 0.7 to 8.0 % by weight.

In one embodiment the mineral wool product is a mineral wool insulation product, such as a mineral wool thermal or acoustical insulation product.

In one embodiment the mineral wool product is a horticultural growing media.

Method of producing a mineral wool product

The present invention also provides a method for producing a mineral wool product by binding mineral fibres with the binder composition.

A particular advantage of the mineral wool product according to the present invention is that it does not require high temperatures for curing. This does not only save energy, reduces VOC and obviates the need for machinery to be highly temperature resistant, but also allows for a high flexibility in a process for the production of mineral wool products with these binders.

In one embodiment the method comprises the steps of:

- making a melt of raw materials,
- fibrerising the melt by means of a fiber forming apparatus to form mineral fibres,
- providing the mineral fibres in the form of a collected web,
- mixing the binder with the mineral fibres before, during or after the provision of the collected web to form a mixture of mineral fibres and binder,
- curing the mixture of mineral fibres and binder.

In one embodiment, the binder is supplied in the close vicinity of the fibre forming apparatus, such as a cup spinning apparatus or a cascade spinning apparatus, in either case immediately after the fibre formation. The fibres with applied binder are thereafter conveyed onto a conveyor belt as a web.

The web may be subjected to longitudinal or length compression after the fibre formation and before substantial curing has taken place.

Fiber forming apparatus

There are various types of centrifugal spinners for fiberising mineral melts.

A conventional centrifugal spinner is a cascade spinner which comprises a sequence of a top (or first) rotor and a subsequent (or second) rotor and optionally other subsequent rotors (such as third and fourth rotors). Each rotor rotates about a different substantially horizontal axis with a rotational direction opposite to the rotational direction of the or each adjacent rotor in the sequence. The different horizontal axes are arranged such that melt which is poured on to the top rotor is thrown in sequence on to the peripheral surface of the or each subsequent rotor, and fibres are thrown off the or each subsequent rotor, and optionally also off the top rotor.

In one embodiment, a cascade spinner or other spinner is arranged to fiberise the melt and the fibres are entrained in air as a cloud of the fibres.

Many fiber forming apparatuses comprise a disc or cup that spins around a substantially vertical axis. It is then conventional to arrange several of these spinners in-line, i.e. substantially in the first direction, for instance as described in GB-A-926,749, US-A-3,824,086 and WO-A-83/03092.

There is usually a stream of air associated with the one or each fiberising rotor whereby the fibres are entrained in this air as they are formed off the surface of the rotor.

In one embodiment, binder and/or additives is added to the cloud of fibres by known means. The amount of binder and/or additive may be the same for each spinner or it may be different.

In one embodiment, a hydrocarbon oil may be added into the cloud of fibres.

As used herein, the term "collected web" is intended to include any mineral fibres that have been collected together on a surface, i.e. they are no longer entrained in air, e.g. the fibrerised mineral fibres, granulate, tufts or recycled web waste. The collected web could be a primary web that has been formed by collection of fibres on a conveyor belt and provided as a starting material without having been cross-lapped or otherwise consolidated.

Alternatively, the collected web could be a secondary web that has been formed by crosslapping or otherwise consolidating a primary web. Preferably, the collected web is a primary web.

In one embodiment the mixing of the binder with the mineral fibres is done after the provision of the collected web in the following steps:

- subjecting the collected web of mineral fibres to a disentanglement process,
- suspending the mineral fibres in a primary air flow,
- mixing binder composition with the mineral fibres before, during or after the disentanglement process to form a mixture of mineral fibres and binder.

A method of producing a mineral wool product comprising the process step of disentanglement is described in EP10190521.

In one embodiment, the disentanglement process comprises feeding the collected web of mineral fibres from a duct with a lower relative air flow to a duct with a higher relative air flow. In this embodiment, the disentanglement is believed to occur, because the fibres that enter the duct with the higher relative air flow first are dragged away from the subsequent fibres in the web. This type of disentanglement is particularly effective for producing open tufts of fibres, rather than the compacted lumps that can result in an uneven distribution of materials in the product.

According to a particularly preferred embodiment, the disentanglement process comprises feeding the collected web to at least one roller which rotates about its longitudinal axis and has spikes protruding from its circumferential surface. In this embodiment, the rotating roller will usually also contribute at least in part to the higher relative air flow.

Often, rotation of the roller is the sole source of the higher relative air flow.

In preferred embodiments, the mineral fibres and optionally the binder are fed to the roller from above. It is also preferred for the disentangled mineral fibres and optionally the binder to be thrown away from the roller laterally from the lower part of its circumference. In the most preferred embodiment, the mineral fibres are carried approximately 180 degrees by the roller before being thrown off.

The binder may be mixed with the mineral fibres before, during or after the disentanglement process. In some embodiments, it is preferred to mix the binder with the fibres prior to the disentanglement process. In particular, the fibres can be in the form of an uncured collected web containing binder.

It is also feasible that the binder be pre-mixed with a collected web of mineral fibres before the disentanglement process. Further mixing could occur during and after the disentanglement process. Alternatively, it could be supplied to the primary air flow separately and mixed in the primary air flow.

The mixture of mineral fibres and binder is collected from the primary air flow by any suitable means. In one embodiment, the primary air flow is directed into the top of a cyclone chamber, which is open at its lower end and the mixture is collected from the lower end of the cyclone chamber.

The mixture of mineral fibres and binder is preferably thrown from the disentanglement process into a forming chamber.

Having undergone the disentanglement process, the mixture of mineral fibres and binder is collected, pressed and cured. Preferably, the mixture is collected on a foraminous conveyor belt having suction means positioned below it.

In a preferred method according to the invention, the mixture of binder and mineral fibres, having been collected, is pressed and cured.

In a preferred method according to the invention, the mixture of binder and mineral fibres, having been collected, is scalped before being pressed and cured.

The method may be performed as a batch process, however according to an embodiment the method is performed at a mineral wool production line feeding a primary or secondary mineral wool web into the fibre separating process, which provides a particularly cost efficient and versatile method to provide composites having favourable mechanical properties and thermal insulation properties in a wide range of densities.

At the same time, because of the curing at ambient temperature, the likelihood of uncured binder spots is strongly decreased.

Curing

The web is cured by a chemical and/or physical reaction of the binder components.

In one embodiment, the curing takes place in a curing device.

The curing is carried out at temperatures from 5 to 95 °C, such as 10 to 60 °C, such as 20 to 40 °C.

The curing process may commence immediately after application of the binder to the fibres. The curing is defined as a process whereby the binder composition undergoes a chemical reaction which usually increases the molecular weight of the compounds in the binder composition and thereby increases the viscosity of the binder composition, usually until the binder composition reaches a solid state.

In one embodiment the curing process comprises cross-linking and/or water inclusion as crystal water.

In one embodiment the cured binder contains crystal water that may decrease in content and raise in content depending on the prevailing conditions of temperature, pressure and humidity.

In one embodiment the curing takes place in a conventional curing oven for mineral wool production operating at a temperature of from 5 to 95 °C, such as 10 to 60 °C, such as 20 to 40 °C.

In one embodiment the curing process comprises a drying process.

In a preferred embodiment, the curing of the binder in contact with the mineral fibers takes place in a heat press.

The curing of a binder in contact with the mineral fibers in a heat press has the particular advantage that it enables the production of high-density products.

In one embodiment the curing process comprises drying by pressure. The pressure may be applied by blowing air or gas to the mixture of mineral fibres and binder. The blowing process may be accompanied by heating or cooling or it may be at ambient temperature.

In one embodiment the curing process takes place in a humid environment.

The humid environment may have a relative humidity RH of 60-99%, such as 70-95%, such as 80-92%. The curing in a humid environment may be followed by curing or drying to obtain a state of the prevalent humidity.

The mineral wool product can be in any conventional configuration, for instance a mat or slab, and can be cut and/or shaped (e.g. into pipe sections) before, during or after curing of the binder.

Advantages of the binder composition

The mineral wool product according to the present invention has the surprising advantage that it can be produced by a very simple binder which requires as little as only two components, namely at least one protein and at least one phenol containing compound. The mineral wool product according to the present invention is therefore produced from natural and non-toxic components and is therefore safe to work with. At the same time, the mineral wool product according to the present invention is produced from a binder based on renewable resources.

A further advantage is the possibility of curing at ambient temperature or in the vicinity of ambient temperature. This not only leads to savings of energy consumption and less complexity of the machinery required but also decreases the likelihood of uncured binder spots, which can occur during thermal curing of conventional binders.

A further advantage is the strongly reduced punking risk.

Punking may be associated with exothermic reactions during manufacturing of the mineral wool product which increase temperatures through the thickness of the insulation causing a fusing or devitrification of the mineral fibres and eventually creating a fire hazard. In the worst case, punking causes fires in the stacked pallets stored in warehouses or during transportation.

Yet another advantage is the absence of emissions during curing, in particular the absence of VOC emissions.

Further important advantages are the self-repair capacities of mineral wool products produced from the binders.

A further advantage of the mineral wool products according to the present invention produced with the binder described is that they may be shaped as desired after application of the binder but prior to curing. This opens the possibility for making tailor-made products, like pipe sections.

## Examples

In the following examples, several binders which fall under the definition of the present invention for the binder used in the mineral wool product according to the invention were prepared and compared to binders according to the prior art.

Binders according to the prior art

The following properties were determined for the binders according the prior art.

*Reagents*

50% aq. hypophosphorous acid and 28% aq. ammonia were supplied by Sigma Aldrich. D-(+)-glucose monohydrate was supplied by Merck. 75.1 % aq. glucose syrup with a DE-value of 95 to less than 100 (C*sweet D 02767 ex Cargill) was supplied by Cargill. Silane (Momentive VS-142) was supplied by Momentive and was calculated as 100% for simplicity. All other components were supplied in high purity by Sigma-Aldrich and were assumed anhydrous for simplicity.

*Binder component solids content - definition*

The content of each of the components in a given binder solution before curing is based on the anhydrous mass of the components. The following formula can be used:

$$Binder\ component\ solids\ content\ (\%) = \frac{binder\ component\ A\ solids\ (g) + binder\ component\ B\ solids\ (g) + \cdots}{total\ weight\ of\ mixture\ (g)} \times 100\%$$

*Binder solids - definition and procedure*

The content of binder after curing is termed "binder solids".

Disc-shaped stone wool samples (diameter: 5 cm; height 1 cm) were cut out of stone wool and heat-treated at 580 °C for at least 30 minutes to remove all organics. The solids of the binder mixture (see below for mixing examples) were measured by distributing a sample of the binder mixture (approx. 2 g) onto a heat treated stone wool disc in a tin foil container. The weight of the tin foil container containing the stone wool disc was weighed before and directly after addition of the binder mixture. Two such binder mixture loaded stone wool discs in tin foil containers were produced and they were then heated at 200 °C for 1 hour. After cooling and storing at room temperature for 10 minutes, the samples were weighed and the binder solids was calculated as an average of the two results. A binder with the desired binder solids could then be produced by diluting with the required amount of water and 10% aq. silane (Momentive VS-142).

*Reaction loss - definition*

The reaction loss is defined as the difference between the binder component solids content and the binder solids.

*Mechanical strength studies (tablet tests) - procedure*

The mechanical strength of the binders was tested in a tablet test. For each binder, six tablets were manufactured from a mixture of the binder and stone wool shots from the stone wool spinning production. The shots are particles which have the same melt composition as the stone wool fibers, and the shots are normally considered a waste product from the spinning process. The shots used for the tablet composition have a size of 0.25-0.50 mm.

A 15% binder solids binder solution containing 0.5% silane (Momentive VS-142) of binder solids was obtained as described above under "binder solids". A sample of this binder solution (4.0 g) was mixed well with shots (20.0 g). The resulting mixture was then transferred into a round aluminum foil container (bottom Ø = 4.5 cm, top Ø = 7.5 cm, height = 1.5 cm). The mixture was then pressed hard with a suitably sized flat bottom glass or plastic beaker to generate an even tablet surface. Six tablets from each binder were made in this fashion. The resulting tablets were then cured at 250 °C for 1 h. After cooling to room temperature, the tablets were carefully taken out of the containers. Three of the tablets were aged in a water bath at 80 °C for 3 h.

After drying for 1-2 days, all tablets were then broken in a 3 point bending test (test speed: 10.0 mm/min; rupture level: 50%; nominal strength: 30N/mm$^2$; support distance: 40 mm; max deflection 20 mm; nominal e-module 10000 N/mm$^2$) on a Bent Tram machine to investigate their mechanical strengths. The tablets were placed with the "bottom face" up (i.e. the face with Ø = 4.5 cm) in the machine.

*Theoretical binder content in tablets - definition*

The theoretical binder content in tablets is calculated by dividing the binder solids used in the tablet production with the sum of the binder solids and shots used in the tablet production. The following formula can be used:

$$Theoretical\ binder\ content\ (\%) = \frac{binder\ mixture\ used\ (g) \times binder\ solids\ (\%)}{binder\ mixture\ used\ (g) \times binder\ solids\ (\%) + shots\ used\ (g)} \times 100\%$$

For each binder mixture, six tablets were produced. The theoretical binder content for each binder tested was calculated as an average of these six tablets.

Reference binders from the prior art prepared as comparative examples

*Binder example, reference binder A*

A mixture of anhydrous citric acid (10.2 g, 53.1 mmol) and D-(+)-glucose monohydrate (57.3 g; thus efficiently 52.1 g dextrose) in water (157.5 g) was stirred at room temperature until a clear solution was obtained. 28% aq. ammonia (7.80 g; thus efficiently 2.16 g, 128.4 mmol ammonia) was then added dropwise. The binder solids were then measured (17.4%). For mechanical strength studies (15% binder solids solution, 0.5% silane of binder solids), the binder mixture was diluted with water (0.149 g / g binder mixture) and 10% aq. silane (0.009 g / g binder mixture, Momentive VS-142). The final binder mixture had pH = 5.1.

*Binder example, reference binder B (phenol-formaldehyde resin modified with urea, a PUF-resol)*

A phenol-formaldehyde resin is prepared by reacting 37% aq. formaldehyde (606 g) and phenol (189 g) in the presence of 46% aq. potassium hydroxide (25.5 g) at a reaction temperature of 84°C preceded by a heating rate of approximately 1°C per minute. The reaction is continued at 84 °C until the acid tolerance of the resin is 4 and most of the phenol is converted. Urea (241 g) is then added and the mixture is cooled.

The acid tolerance (AT) expresses the number of times a given volume of a binder can be diluted with acid without the mixture becoming cloudy (the binder precipitates). Sulfuric acid is used to determine the stop criterion in a binder production and an acid tolerance lower than 4 indicates the end of the binder reaction. To measure the AT, a titrant is produced from diluting 2.5 ml conc. sulfuric acid (>99 %) with 1 L ion exchanged water. 5 mL of the binder to be investigated is then titrated at room temperature with this titrant while keeping the binder in motion by manually shaking it; if preferred, use a magnetic stirrer and a magnetic stick. Titration is continued until a slight cloud appears in the binder, which does not disappear when the binder is shaken.

The acid tolerance (AT) is calculated by dividing the amount of acid used for the titration (mL) with the amount of sample (mL):

$$AT = (Used\ titration\ volume\ (mL)) / (Sample\ volume\ (mL))$$

Using the urea-modified phenol-formaldehyde resin obtained, a binder is made by addition of 25% aq. ammonia (90 mL) and ammonium sulfate (13.2 g) followed by water (1.30 kg). The binder solids were then measured as described above and the mixture was diluted with the required amount of water and silane (Momentive VS-142) for mechanical strength studies (15% binder solids solution, 0.5% silane of binder solids).

*Binder example, reference binder C*

A mixture of L-ascorbic acid (1.50 g, 8.52 mmol) and 75.1% aq. glucose syrup (18.0 g; thus efficiently 13.5 g glucose syrup) in water (30.5 g) was stirred at room temperature until a clear solution was obtained. 50% aq. hypophosphorous acid (0.60 g; thus efficiently 0.30 g, 4.55 mmol hypophosphorous acid) and urea (0.75 g) were then added. 28% aq. ammonia (0.99 g; thus efficiently 0.28 g, 16.3 mmol ammonia) was then added dropwise until pH = 6.9. The binder solids were then measured (21.5%). For mechanical strength studies (15% binder solids solution, 0.5% silane of binder solids), the binder mixture was diluted with water (0.423 g / g binder mixture) and 10% aq. silane (0.011 g / g binder mixture, Momentive VS-142). The final binder mixture had pH = 7.0.

*Binder example, reference binder D*

A mixture of 75.1% aq. glucose syrup (60.0 g; thus efficiently 45.0 g glucose syrup), ammonium sulfamate (2.25 g, 19.7 mmol) and urea (2.25 g) in water (105.1 g) was stirred at room temperature until a clear solution was obtained. 28% aq. ammonia (0.12 g; thus efficiently 0.03 g, 1.97 mmol ammonia) was then added dropwise until pH = 8.2. The binder solids were then measured (21.6%). For mechanical strength studies (15% binder solids solution, 0.5% silane of binder solids), the binder mixture was diluted with water (0.432 g / g binder mixture) and 10% aq. silane (0.011 g / g binder mixture, Momentive VS-142). The final binder mixture had pH = 8.2.

*Binder example, reference binder E (based on alkanolamine-polycarboxylic acid anhydride reaction products)*

Diethanolamine (DEA, 231.4 g) is placed in a 5-litre glass reactor provided with a stirrer and a heating/cooling jacket. The temperature of the diethanolamine is raised to 60 °C where after tetrahydrophthalic anhydride (THPA, 128.9 g) is added. After raising the temperature and keeping it at 130 °C, a second portion of tetrahydrophthalic anhydride (64.5 g) is added followed by trimellitic anhydride (TMA, 128.9 g). After reacting at 130 °C for 1 hour, the mixture is cooled to 95 °C. Water (190.8 g) is added and stirring is continued for 1 hour. After cooling to ambient temperature, the mixture is poured into water (3.40 kg) and 50% aq. hypophosphorous acid (9.6 g) and 25% aq. ammonia (107.9 g) are added under stirring. Glucose syrup (1.11 kg) is heated to 60 °C and then added under stirring followed by 50% aq. silane (5.0 g, Momentive VS-142). The binder solids were then measured as described above and the mixture was diluted with the required amount of water for mechanical strength measurements (15% binder solids solutions).

*Binder example, reference binder F (based on alkanolamine-polycarboxylic acid anhydride reaction products)*

Diethanolamine (DEA, 120.5 g) is placed in a 5-litre glass reactor provided with a stirrer and a heating/cooling jacket. The temperature of the diethanolamine is raised to 60 °C where after tetrahydrophthalic anhydride (THPA, 67.1 g) is added. After raising the temperature and keeping it at 130 °C, a second portion of tetrahydrophthalic anhydride (33.6 g) is added followed by trimellitic anhydride (TMA, 67.1 g). After reacting at 130 °C for 1 hour, the mixture is cooled to 95 °C. Water (241.7 g) is added and stirring is continued for 1 hour. Urea (216.1 g) is then added and stirring is continued until all solids are dissolved. After cooling to ambient temperature, the mixture is poured into water (3.32 kg) and 50% aq. hypophosphorous acid (5.0 g) and 25% aq. ammonia (56.3 g) are added under stirring. Glucose syrup (1.24 kg) is heated to 60 °C and then added under stirring followed by 50% aq. silane (5.0 g, Momentive VS-142). The binder solids were then measured as described above and the mixture was diluted with the required amount of water for mechanical strength measurements (15% binder solids solutions).

Binders for the mineral wool product according to the present invention

The following properties were determined for the binders for the mineral wool product according the present invention.

*Reagents*

Medium gel strength gelatin from porcine skin (170-195 g Bloom), tannic acid, sodium hydroxide and potassium hydroxide were obtained from Sigma-Aldrich. For simplicity, these reagents were considered completely pure and anhydrous.

*Binder component solids content - definition*

The content of each of the components in a given binder solution before curing is based on the anhydrous mass of the components. The following formula can be used:

$$Binder\ component\ solids\ content\ (\%) = \frac{binder\ component\ A\ solids\ (g) + binder\ component\ B\ solids\ (g) + \cdots}{total\ weight\ of\ mixture\ (g)} \times 100\%$$

The binder examples listed below were mixed to a binder component solids content of $15 \pm 1\%$ for convenience.

*Mechanical strength studies (tablet tests) - procedure*

The mechanical strength of the binders was tested in a tablet test. For each binder, six tablets were manufactured from a mixture of the binder and stone wool shots from the stone wool spinning production. The shots are particles which have the same melt composition as the stone wool fibers, and the shots are normally considered a waste product from the spinning process. The shots used for the tablet composition have a size of 0.25-0.50 mm.

A binder mixture containing $15 \pm 1\%$ binder component solids was obtained as described in the examples below. A sample of this binder solution (4.0 g) was mixed well with shots (20.0 g). The resulting mixture was then transferred into a round aluminum foil container (bottom $\varnothing$ = 4.5 cm, top $\varnothing$ = 7.5 cm, height = 1.5 cm). The mixture was spread out evenly in the container using a broad spatula, generating an even tablet surface. Six tablets from each binder were made in this fashion.

Containers with tablets made with binders that were intended to cure at room temperature were left at room temperature for two days. Containers with tablets that were intended to cure below room temperature were first left at 11 °C for one day followed by two days at room temperature. Containers with tablets that were intended to cure above room temperature were left at 41 °C, 61 °C or 81 °C for 5 h, 2 h, or 1 h, respectively, followed by one day at room temperature.

The tablets were then carefully taken out of the containers. Three of the tablets were aged in a water bath at 80 °C for 3 h.

After drying for two days, all tablets were then broken in a 3 point bending test (test speed: 10.0 mm/min; rupture level: 50%; nominal strength: 30 N/mm$^2$; support distance: 40 mm; max deflection 20 mm; nominal e-module 10000 N/mm$^2$) on a Bent Tram machine to investigate their mechanical strengths. The tablets were placed with the "bottom face" up (i.e. the face with $\varnothing$ = 4.5 cm) in the machine.

*Theoretical binder content in tablets - definition*

The theoretical binder content in tablets is calculated by dividing the binder component solids used in the tablet production with the sum of the binder component solids and shots used in the tablet production. The following formula can be used:

$$Theoretical\ binder\ content\ (\%) = \frac{binder\ mixture\ used\ (g) \times binder\ comp.\ solids\ content\ (\%)}{binder\ mixture\ used\ (g) \times binder\ comp.\ solids\ content\ (\%) + shots\ used\ (g)} \times 100\%$$

For each binder mixture, six tablets were produced. The theoretical binder content for each binder tested was calculated as an average of these six tablets.

*Measured binder content in tablets - definition*

The measured binder content in tablets is calculated by dividing the weight of the dried tablet minus the shots in the tablet with the weight of the dried tablet. The following calculation can be used:

$$Meas.\,binder\,content\,(\%) = \frac{dry\,tablet\,(g) - freshly\,made\,tablet\,(g) \times \frac{shots\,used\,(g)}{shots\,used\,(g) + binder\,mixture\,used\,(g)}}{dry\,tablet\,(g)} \times 100\%$$

For each binder mixture, six tablets were produced. The measured binder content for each binder tested was calculated as an average of these six tablets.

*Film tests - procedure*

A binder mixture containing $15 \pm 1\%$ binder component solids was obtained as described in the examples below. A sample large enough to cover the bottom with a layer of approx. 1-2 mm thickness was then transferred into a round aluminum foil container (bottom $\emptyset$ = 4.5 cm, top $\emptyset$ = 7.5 cm, height = 1.5 cm). For each binder mixture, two such samples were produced and they were then left at room temperature for two days.
The generated films were carefully taken out of the container and one of the films was tested for water resistance by submerging the film halfway into water at 80 °C. Binder compositions with no water resistance would dissolve quickly in this test.

Binder compositions for the mineral wool product according to the present invention

For the preparation of the binder composition for the mineral wool product according to the present invention, three mixing methods where employed. These three mixing methods are described in the following:
In "Method A" a solution of the gelatin was first basified with aq. NaOH whereupon tannic acid was added. The pH of the resulting mixture was kept at >8 by addition of further aq. NaOH in portions.
In "Method B", a freshly made or one day old pre-mixed tannic acid-NaOH solution (10% tannic acid in 1M NaOH) was added to the solution of the gelatin. The pre-mixed tannic acid-NaOH solution contained sufficient NaOH to bring the final mixture to pH > 8.
In "Method C", the freshly made pre-mixed tannic acid-NaOH solution (14.3% tannic acid in 1M NaOH) contained less NaOH and the gelatin solution was therefore basified to pH >8 with aq. NaOH before addition of the tannic acid-NaOH solution

*Binder example, entry 1*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). 1M NaOH (3.10 g) was then added (pH 8.8) and the resulting solution was stirred for 30 minutes further at 50 °C before being used in the subsequent experiments.

*Binder example, entry 5*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). The solution was stirred for 30 minutes further at 50 °C before being used in the subsequent experiments.

*Binder example, entry 7*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). 1M NaOH (2.99 g) was then added (pH 9.1) followed by tannic acid (0.50 g). The mixture was stirred vigorously for 30 minutes at 50 °C while keeping pH > 8.5 by portion-wise addition of further 1M NaOH (3.74 g). The resulting brown mixture (pH 9.2) was then used in the subsequent experiments.

*Binder example, entry 8*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). 1M NaOH (3.12 g) was then added (pH 9.1) followed by tannic acid (1.0 g). The mixture was stirred vigorously for 30 minutes at 50 °C while keeping pH > 8.5 by portion-wise addition of further 1M NaOH (6.46 g). The resulting brown mixture (pH 9.0) was then used in the subsequent experiments.

*Binder example, entry 9*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.1). 1M NaOH (4.93 g) was then added (pH 9.9) followed by tannic acid (2.0 g). The mixture was stirred vigorously for 30 minutes at 50 °C while keeping pH > 8.5 by portion-wise addition of further 1M NaOH (8.99 g). The resulting brown mixture (pH 8.6) was then used in the subsequent experiments.

*Binder example, entry 12*

Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 5.2). 1M KOH (3.47 g) was then added (pH 9.1) followed by tannic acid (1.0 g). The mixture was stirred vigorously for 30 minutes at 50 °C while keeping pH > 8.5 by portion-wise addition of further 1M KOH (7.36 g). The resulting brown mixture (pH 8.8) was then used in the subsequent experiments.

*Binder example, entry 17*

To 1M NaOH (18.0 g) at room temperature was added tannic acid (2.0 g). The resulting mixture was stirred for 15 minutes after which time a brown-greenish solution was obtained.
Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 4.9). Tannic acid in aq. NaOH (10.0 g, produced as above) was then added (pH 9.0). The mixture was stirred vigorously for 30 minutes at 50 °C and resulting brown mixture (pH 8.5) was then used in the subsequent experiments.

*Binder example, entry 19*

To 1M NaOH (12.0 g) at room temperature was added tannic acid (2.0 g). The resulting mixture was stirred for 15 minutes after which time a brown-greenish solution was obtained.
Gelatin from porcine skin, medium gel strength (10.0 g) was swelled in water (56.7 g) for 30 min at room temperature. The mixture was then placed in a water bath at 50 °C and stirred a few minutes until a clear solution was obtained (pH 4.9). 1M NaOH (3.00 g) was then added (pH 8.9) followed by tannic acid in aq. NaOH (7.0 g, produced as above). The mixture was stirred vigorously for 30 minutes at 50 °C and resulting brown mixture (pH 8.7) was then used in the subsequent experiments.
The other binders mentioned in Table 1 were prepared in a manner analogous to the preparations described above.

TABLE 1-1

| Example | Reference binders | | | | | |
|---|---|---|---|---|---|---|
| | A | B | C | D | E | F |
| **Binder composition** | | | | | | |
| Acid or Ascorb. acid (%-wt.) | | | | | | |
| L-Ascorbic acid | - | - | 10 | - | - | - |
| Citric acid | 16 | - | - | - | - | - |
| Carbohydrate (%-wt.) | | | | | | |

(continued)

| Example | Reference binders | | | | | |
|---|---|---|---|---|---|---|
|  | A | B | C | D | E | F |
| **Binder composition** |  |  |  |  |  |  |
| Glucose syrup | - | - | 90 | 100 | - | - |
| Glucose | 84 | - | - | - | - | - |
| Additive (%-wt.)[a] |  |  |  |  |  |  |
| Hypophosphorous acid | - | - | 2 | - | - | - |
| Ammonium sulfamate | - | - | - | 5 | - | - |
| Urea | - | - | 5 | 5 | - | - |
| Amine (equiv.) [b] |  |  |  |  |  |  |
| Ammonia (added) | 0.8 | - | 1.2 | 0.1 | - | - |
| Silane (% of binder solids) | 0.5 | 0.5 | 0.5 | 0.5 | - | - |
|  |  |  |  |  |  |  |
| **Binder properties** |  |  |  |  |  |  |
| Binder component solids content (%) | - | - | 21.8 | 20.2 | 21.1 | 21.6 |
| Reaction loss (%) | 37.3 | 28.5 | 31.1 | 25.9 | 28.9 | 30.6 |
| Binder solids (%) | 15.0 | 15.0 | 15.0 | 15.0 | 15.0 | 15.0 |
| pH of binder mixture | 5.1 | 9.6 | 7.0 | 8.2 | 6.1 | 6.2 |
|  |  |  |  |  |  |  |
| **Curing conditions** |  |  |  |  |  |  |
| Temperature (°C) | 250 | 250 | 250 | 250 | 250 | 250 |
|  |  |  |  |  |  |  |
| **Tablet properties** |  |  |  |  |  |  |
| Theoretical binder content in tablets (%)[c] | 2.9 | 2.9 | 2.9 | 2.9 | 2.9 | 2.9 |
| Mechanical strength, unaged (kN) | 0.17 | 0.16 | 0.16 | 0.16 | 0.20 | 0.09 |
| Mechanical strength, aged (kN) | 0.13 | 0.09 | 0.08 | 0.09 | 0.16 | 0.07 |
| [a] Of Carbohydrate + ascorbic acid. [b] Molar amine equivalents relative to molar mineral or organic acid equivalents. [c] Based on binder solids. | | | | | | |

TABLE 1-2

| Example | Gelatin, various conditions (no phenol containing compound) | | | | |
|---|---|---|---|---|---|
|  | 1 | 2 | 3 | 4 | 5 |
| **Binder composition** |  |  |  |  |  |
| Protein (%-wt.) |  |  |  |  |  |
| Gelatin (porcine skin), medium gel strength | 100 | 100 | 100 | 100 | 100 |
| Phenol and/or quinone containing compound (%-wt.) [a] |  |  |  |  |  |
| Tannic acid | - | - | - | - | - |

(continued)

| | Gelatin, various conditions (no phenol containing compound) | | | | |
|---|---|---|---|---|---|
| **Example** | 1 | 2 | 3 | 4 | 5 |
| **Binder composition** | | | | | |
| Base (%-wt.) [b] | | | | | |
| Sodium hydroxide | 1.2 | 1.0 | 1.0 | 1.0 | - |
| Potassium hydroxide | - | - | - | - | - |
| | | | | | |
| **Binder properties** | | | | | |
| Mixing method | - | - | - | - | - |
| Binder component solids content (%) | 14.5 | 14.6 | 14.6 | 14.6 | 15.0 |
| pH of binder mixture | 8.8 | 8.6 | 8.6 | 8.6 | 5.1 |
| | | | | | |
| **Curing conditions** | | | | | |
| Temperature (°C) | rt | 43 | 61 | 81 | rt |
| | | | | | |
| **Tablet properties** | | | | | |
| Theoretical binder content in tablets (%)[c] | 2.8 | 2.8 | 2.8 | 2.8 | 2.9 |
| Measured binder content in tablets (%) | 3.8 | 3.9 | 3.9 | 3.8 | 3.9 |
| Mechanical strength unaged (kN) | 0.23 | 0.27 | 0.25 | 0.26 | 0.24 |
| Mechanical strength, aged (kN) | 0[d] | 0[d] | 0[d] | 0[d] | 0[d] |
| | | | | | |
| **Film properties** | | | | | |
| Stable in ageing test | No | - | - | - | No |

[a] Of gelatin. [b] Of gelatin + tannic acid. [c] Based on binder component solids content. [d] Complete disintegration of the tablets was observed during the ageing treatment.

TABLE 1-3

| | Gelatin, tannic acid sodium hydroxide or potassium hydroxide, rt Method A | | | | | | |
|---|---|---|---|---|---|---|---|
| **Example** | 6 | 7 | 8 | 9 | 10 | 11 | 12 |
| **Binder composition** | | | | | | | |
| Protein (%-wt.) | | | | | | | |
| Gelatin (porcine skin), medium gel strength | 100 | 100 | 100 | 100 | 100 | 100 | 100 |
| Phenol containing compound (%-wt.) [a] | | | | | | | |
| Tannic acid | 2 | 5 | 10 | 20 | 30 | 50 | 10 |
| Base (%-wt.) [b] | | | | | | | |

(continued)

| | Gelatin, tannic acid sodium hydroxide or potassium hydroxide, rt Method A | | | | | | |
|---|---|---|---|---|---|---|---|
| **Example** | 6 | 7 | 8 | 9 | 10 | 11 | 12 |
| **Binder composition** | | | | | | | |
| Sodium hydroxide | 1.7 | 2.5 | 3.3 | 4.5 | 6.4 | 8.5 | - |
| Potassium hydroxide | - | - | - | - | - | - | 5.2 |
| | | | | | | | |
| **Binder properties** | | | | | | | |
| Mixing method | A | A | A | A | A | A | A |
| Binder component solids content (%) | 14.6 | 14.6 | 14.7 | 15.2 | 15.1 | 15.5 | 14.7 |
| pH of binder mixture | 8.8 | 9.2 | 9.0 | 8.6 | 8.8 | 8.8 | 8.8 |
| | | | | | | | |
| **Curing conditions** | | | | | | | |
| Temperature (°C) | rt | rt | rt | rt | rt | rt | rt |
| | | | | | | | |
| **Tablet properties** | | | | | | | |
| Theoretical binder content in tablets (%)[c] | 2.8 | 2.8 | 2.9 | 2.9 | 2.9 | 3.0 | 2.9 |
| Measured binder content in tablets (%) | 3.9 | 3.9 | 4.0 | 4.0 | 4.0 | 3.8 | 3.9 |
| Mechanical strenath, unaged (kN) | 0.28 | 0.30 | 0.27 | 0.26 | 0.15 | 0.18 | 0.27 |
| Mechanical strength, aged (kN) | 0.12 | 0.17 | 0.17 | 0.12 | 0.06 | 0.01 | 0.16 |
| | | | | | | | |
| **Film properties** | | | | | | | |
| Stable in ageing test | Yes | Yes | Yes | Yes | Yes | Yes | Yes |

[a] Of gelatin. [b] Of gelatin + tannic acid. [c] Based on binder component solids content. [d] Complete disintegration of the tablets was observed during the ageing treatment.

TABLE 1-4

| | Gelatin, tannic acid, sodium hydroxide, various temp., Method A | | | | | Gelatin, tannic acid, sodium hydroxide, rt. Methods B and C | | |
|---|---|---|---|---|---|---|---|---|
| **Example** | 13 | 8 | 14 | 15 | 16 | 17 | 18 | 19 |
| **Binder composition** | | | | | | | | |
| Protein (%-wt.) | | | | | | | | |
| Gelatin (porcine skin), medium gel strength | 100 | 100 | 100 | 100 | 100 | 100 | 100 | 100 |
| Phenol containing compound (%-wt.) [a] | | | | | | | | |
| Tannic acid | 10 | 10 | 10 | 10 | 10 | 10 | 10 | 10 |

(continued)

| | Gelatin, tannic acid, sodium hydroxide, various temp., Method A | | | | | Gelatin, tannic acid, sodium hydroxide, rt. Methods B and C | | |
|---|---|---|---|---|---|---|---|---|
| **Example** | 13 | 8 | 14 | 15 | 16 | 17 | 18 | 19 |
| **Binder composition** | | | | | | | | |
| Base (%-wt.) [b] | | | | | | | | |
|     Sodium hydroxide | 4.0 | 3.3 | 3.3 | 3.3 | 3.0 | 3.1 | 3.7 | 3.1 |
|     Potassium hydroxide | - | - | - | - | - | - | - | - |
| | | | | | | | | |
| **Binder properties** | | | | | | | | |
|     Mixing method | A | A | A | A | A | B[c] | B[d] | C[c] |
|     Binder component solids content (%) | 14.5 | 14.7 | 14.7 | 14.8 | 14.9 | 14.8 | 14.5 | 14.8 |
|     pH of binder mixture | 9.5 | 9.0 | 8.9 | 8.9 | 8.7 | 8.5 | 8.5 | 8.7 |
| | | | | | | | | |
| **Curing conditions** | | | | | | | | |
|     Temperature (°C) | 11 | rt | 43 | 61 | 81 | rt | rt | rt |
| | | | | | | | | |
| **Tablet properties** | | | | | | | | |
|     Theoretical binder content in tablets (%)[e] | 2.8 | 2.9 | 2.9 | 2.9 | 2.9 | 2.9 | 2.8 | 2.9 |
|     Measured binder content in tablets (%) | 3.9 | 4.0 | 3.8 | 3.7 | 4.0 | 4.1 | 4.0 | 4.0 |
|     Mechanical strength, unaged (kN) | 0.28 | 0.27 | 0.23 | 0.27 | 0.23 | 0.26 | 0.23 | 0.27 |
|     Mechanical strength, aged (kN) | 0.18 | 0.17 | 0.09 | 0.11 | 0.08 | 0.17 | 0.17 | 0.16 |
| | | | | | | | | |
| **Film properties** | | | | | | | | |
|     Stable in ageing test | - | Yes | - | - | - | Yes | Yes | Yes |

[a] Of gelatin. [b] Of gelatin + tannic acid. [c] Made using freshly prepared solution of tannic acid in aq. sodium hydroxide. [d] Made using one day o solution of tannic acid in aq. sodium hydroxide. [e] Based on binder component solids content.

The following observations and conclusions can be obtained from the experimental work documented in Tables 1-1 to 1-4:

When comparing the theoretical binder content and the measured binding content for the examples of the binder for the mineral wool product according to the present invention in Table 1-3 and Table 1-4, it can be seen that the measured binder content is considerably higher. The applicant believes that this is in part due to the inclusion of considerable amounts of crystal water in the cured binder. Further, due to the curing at very low temperatures, the binders for the mineral wool product according to the present invention do not experience any significant reaction loss. Accordingly, a higher LOI can be achieved with the use of less organic starting material, when compared with other binders based on renewable resources like the reference binders A, C and D.

As can be seen when comparing the results documented in Table 1-3 and Table 1-4 with the results documented in Table 1-1, the mineral wool products according to the present invention can have a higher mechanical strength (aged and unaged) by using the same amount of binder (theoretical binder content) and a much lower curing temperature. This allows a better product to be obtained while at the same time the energy consumption is decreased and a simpler equipment can be used.

**Claims**

1. A mineral wool product comprising mineral fibres bound by a binder resulting from the curing of a formaldehyde-free binder composition for mineral fibres comprising:

   - at least one phenol containing compound,
   - at least one protein,

   wherein the content of phenol containing compound is 1-70 wt.%, such as 2 to 60 wt.%, such as 3 to 50 wt.%, such as 4 to 40 wt.%, such as 5 to 35 wt.%, based on dry protein basis,
   wherein the at least one protein is selected from the group consisting of proteins from animal sources, including collagen, gelatine, hydrolysed gelatine, and protein from milk (casein, whey), eggs, polyphenolic proteins such as mussel foot protein.

2. A mineral wool product according to claim 1, wherein the at least one phenol containing compound comprises a phenol containing compound such as simple phenolics, such as hydroxybenzoic acids, such as hydroxybenzoic aldehydes, such as hydroxyacetophenones, such as hydroxyphenylacetic acids, such as cinnamic acids, such as cinnamic acid esters, such as cinnamyl aldehydes, such as cinnamyl alcohols, such as coumarins, such as isocoumarins, such as chromones, such as flavonoids, such as chalcones, such as dihydrochalcones, such as aurones, such as flavanones, such as flavanonols, such as flavans, such as leucoanthocyanidins, such as flavan-3-ols, such as flavones, such as anthocyanidins, such as deoxyanthocyanidines, such as anthocyanins, such as biflavonyls, such as benzophenones, such as xanthones, such as stilbenes, such as betacyanins, such as polyphenols and/or polyhydroxyphenols, such as lignans, neolignans (dimers or oligomers from coupling of monolignols such as p-coumaryl alcohol, coniferyl alcohol and sinapyl alcohol), such as lignins (synthesized primarily from the monolignol precursors p-coumaryl alcohol, coniferyl alcohol and sinapyl alcohol), such as tannins, such as condensed tannins (proanthocyanidins), such as hydrolysable tannins, such as gallotannins, such as ellagitannins, such as complex tannins, such as tannic acid, such as phlobabenes.

3. A mineral wool product according to claim 2, wherein the tannin is selected from one or more components from the group consisting of tannic acid, condensed tannins (proanthocyanidins), hydrolysable tannins, gallotannins, ellagitannins, complex tannins, and/or tannin originating from one or more of oak, chestnut, staghorn sumac and fringe cups.

4. A mineral wool product according to any one of the above claims, wherein the phenol containing compound comprises one or more synthetic or semisynthetic molecules that contain phenols, polyphenols, quinones, such as a proteins, peptides, peptoids or arylopeptoids modified with phenol containing side chains, such as dendrimers decorated with phenol containing side chains.

5. A mineral wool product according to claim 4, wherein the collagen or gelatin is originating from one or more sources from the group consisting of mammal, bird species, such as from cow, pig, horse, fowl, and/or from scales, skin of fish.

6. A mineral wool product according to any of the above claims, wherein the mass ratio of (lysine + cystein) in the protein to (phenol) in the phenol containing compound is 1:5.78 - 1:0.08, such as 1:2.89 - 1:0.09, such as 1:1.93 - 1:0.12, such as 1:1.45 - 1:0.15, such as 1:1.16 - 1:0.17.

7. A mineral wool product according to any of the above claims, wherein the binder composition further comprises an additive selected from the group of an oxidiser, such as tyrosinase, a metal ion, such as iron ion, a pH-adjuster, preferably in form of a base, such as organic base, such as amine or salts thereof, inorganic bases, such as metal hydroxide, such as KOH or NaOH, ammonia or salts thereof.

8. A mineral wool product according to any of the above claims, wherein the binder composition has a pH of more than 7, such as more than 8, such as more than 9.

9. A mineral wool product according to claim 8, wherein the density of the mineral wool product is in the range of 10-1200 kg/m$^3$, such as 30-800 kg/m$^3$, such as 40-600 kg/m$^3$, such as 50-250 kg/m$^3$, such as 60-200 kg/m$^3$.

**10.** A mineral wool product according to any of claims 8 or 9, wherein the loss on ignition (LOI) is within the range of 0.1 to 25.0 %, such as 0.3 to 18.0 %, such as 0.5 to 12.0 %, such as 0.7 to 8.0 % by weight.

**11.** A method of producing a mineral wool product according to any one of the claims 1 to 7, which comprises the steps of contacting mineral fibres with the binder composition, and curing the binder, wherein the curing is carried out at temperatures from 5 to 95 °C, such as 10 to 60 °C, such as 20 to 40 °C.

**12.** A method of producing a mineral wool product according to claim 11, wherein the method comprises the steps of:

- making a melt of raw materials,
- fibrerising the melt by means of a fibre forming apparatus to form mineral fibres,
- providing the mineral fibres in the form of a collected web,
- mixing the binder composition with the mineral fibres before, during or after the provision of the collected web to form a mixture of mineral fibres and binder composition,
- curing the mixture of mineral fibres and binder composition.

**13.** A method of producing a mineral wool product according to claim 12, wherein the mixing of the binder with the mineral fibres is done after the provision of the collected web in the following steps:

- subjecting the collected web of mineral fibres to a disentanglement process,
- suspending the mineral fibres in a primary air flow,
- mixing the binder with the mineral fibres before, during or after the disentanglement process to form a mixture of mineral fibres and binder.

**14.** A method of producing a mineral wool product according to any of the claims 11-13, wherein the curing process comprises a drying process, in particular by blowing air or gas over the mineral wool product or by increasing temperature.

**Patentansprüche**

**1.** Mineralwollprodukt, umfassend Mineralfasern, die durch ein Bindemittel gebunden sind, das aus dem Härten einer formaldehydfreien Bindemittelzusammensetzung für Mineralfasern resultiert, die umfasst:

- mindestens eine Phenol enthaltende Verbindung,
- mindestens ein Protein,

wobei der Gehalt an Phenol enthaltender Verbindung 1-70 Gew.-%, wie z.B. 2 bis 60 Gew.-%, wie z.B. 3 bis 50 Gew.-%, wie z.B. 4 bis 40 Gew.-%, wie z.B. 5 bis 35 Gew.-%, bezogen auf Trockenproteinbasis, beträgt,
wobei das mindestens eine Protein ausgewählt ist aus der Gruppe bestehend aus Proteinen aus tierischen Quellen, einschließlich Kollagen, Gelatine, hydrolysierter Gelatine, und Proteinen aus Milch (Kasein, Molke), Eiern, polyphenolischen Proteinen, wie z.B. Muschelfußprotein.

**2.** Mineralwollprodukt nach Anspruch 1, wobei die mindestens eine Phenol enthaltende Verbindung eine Phenol enthaltende Verbindung umfasst, wie einfache Phenole, wie Hydroxybenzoesäuren, wie Hydroxybenzoesäurealdehyde, wie Hydroxyacetophenone, wie Hydroxyphenylessigsäuren, wie Zimtsäuren, wie Zimtsäureester, wie Zimtaldehyde, wie Zimtalkohole, wie Cumarine, wie Isocumarine, wie Chromone, wie Flavonoide, wie Chalkone, wie Dihydrochalkone, wie Aurone, wie Flavanone, wie Flavanonole, wie Flavane, wie Leucoanthocyanidine, wie Flavan-3-ole, wie Flavone, wie Anthocyanidine, wie Deoxyanthocyanidine, wie Anthocyanine, wie Biflavonyle, wie Benzophenone, wie Xanthone, wie Stilbene, wie Betacyanine, wie Polyphenole und/oder Polyhydroxyphenole, wie Lignane, Neolignane (Dimere oder Oligomere aus der Kupplung von Monolignolen, wie p-Coumarylalkohol, Coniferylalkohol und Sinapylalkohol), wie Lignine (hauptsächlich aus den Monolignol-Vorstufen p-Coumarylalkohol, Coniferylalkohol und Sinapylalkohol synthetisiert), wie Tannine, wie kondensierte Tannine (Proanthocyanidine), wie hydrolysierbare Tannine, wie Gallotannine, wie Ellagitannine, wie komplexe Tannine, wie Tanninsäure, wie Phlobabene.

**3.** Mineralwollprodukt nach Anspruch 2, wobei das Tannin ausgewählt ist aus einer oder mehreren Komponenten aus der Gruppe bestehend aus Tanninsäure, kondensierten Tanninen (Proanthocyanidinen), hydrolysierbaren Tanninen, Gallotanninen, Ellagitanninen, komplexen Tanninen und/oder Tannin, das aus einer oder mehreren von Eiche, Kastanie, Hirschhornsumach und Fransenbecher stammt.

4. Mineralwollprodukt nach irgendeinem der vorstehenden Ansprüche, wobei die Phenol enthaltende Verbindung ein oder mehrere synthetische oder halbsynthetische Moleküle umfasst, die Phenole, Polyphenole, Chinone, wie z.B. Proteine, Peptide, Peptoide oder Arylpeptoide, die mit Phenol enthaltenden Seitenketten modifiziert sind, wie z.B. mit Phenol enthaltenden Seitenketten ausgestaltete Dendrimere, enthalten.

5. Mineralwollprodukt nach Anspruch 4, wobei das Kollagen oder die Gelatine aus einer oder mehreren Quellen aus der Gruppe bestehend aus Säugetieren, Vogelarten, wie z.B. Rind, Schwein, Pferd, Geflügel und/oder aus Schuppen, Haut von Fischen stammt.

6. Mineralwollprodukt nach irgendeinem der vorstehenden Ansprüche, wobei das Massenverhältnis von (Lysin + Cystein) in dem Protein zu (Phenol) in der Phenol enthaltenden Verbindung 1:5,78 - 1:0,08, wie z.B. 1:2,89 - 1:0,09, wie z.B. 1:1,93 - 1:0,12, wie z.B. 1:1,45 - 1:0,15, wie z.B. 1:1,16 - 1:0,17 beträgt.

7. Mineralwollprodukt nach irgendeinem der vorstehenden Ansprüche, wobei die Bindemittelzusammensetzung ferner ein Additiv umfasst, das ausgewählt ist aus der Gruppe bestehend aus einem Oxidationsmittel, wie z.B. Tyrosinase, einem Metallion, wie z.B. Eisenion, einem pH-Einstellmittel, vorzugsweise in Form einer Base, wie z.B. organische Base, wie z.B. Amin oder Salze davon, anorganische Basen, wie z.B. Metallhydroxid, wie z.B. KOH oder NaOH, Ammoniak oder Salze davon.

8. Mineralwollprodukt nach irgendeinem der vorstehenden Ansprüche, wobei die Bindemittelzusammensetzung einen pH-Wert von mehr als 7, wie z.B. mehr als 8, wie z.B. mehr als 9, aufweist.

9. Mineralwollprodukt nach Anspruch 8, wobei die Dichte des Mineralwollprodukts im Bereich von 10-1200 kg/m$^3$, wie z.B. 30-800 kg/m$^3$, wie z.B. 40-600 kg/m$^3$, wie z.B. 50-250 kg/m$^3$, wie z.B. 60-200 kg/m$^3$, liegt.

10. Mineralwollprodukt nach irgendeinem der Ansprüche 8 oder 9, wobei der Glühverlust (LOI) im Bereich von 0,1 bis 25,0 Gew.-%, wie z.B. 0,3 bis 18,0 Gew.-%, wie z.B. 0,5 bis 12,0 Gew.-%, wie z.B. 0,7 bis 8,0 Gew.-%, liegt.

11. Verfahren zur Herstellung eines Mineralwollprodukts nach irgendeinem der Ansprüche 1 bis 7, das die Schritte des Inkontaktbringens von Mineralfasern mit der Bindemittelzusammensetzung und des Härtens des Bindemittels umfasst, wobei das Härten bei Temperaturen von 5 bis 95 °C, wie z.B. 10 bis 60 °C, wie z.B. 20 bis 40 °C, durchgeführt wird.

12. Verfahren zur Herstellung eines Mineralwollprodukts nach Anspruch 11, wobei das Verfahren die folgenden Schritte umfasst:

- Erstellen einer Schmelze von Rohstoffen,
- Zerfasern der Schmelze mit Hilfe einer Faserbildungsvorrichtung zur Bildung von Mineralfasern,
- Bereitstellen der Mineralfasern in Form einer gesammelten Bahn,
- Mischen der Bindemittelzusammensetzung mit den Mineralfasern vor, während oder nach der Bereitstellung der gesammelten Bahn, um eine Mischung aus Mineralfasern und Bindemittelzusammensetzung zu bilden,
- Härten der Mischung aus Mineralfasern und Bindemittelzusammensetzung.

13. Verfahren zur Herstellung eines Mineralwollprodukts nach Anspruch 12, wobei das Mischen des Bindemittels mit den Mineralfasern nach der Bereitstellung der gesammelten Bahn in den folgenden Schritten erfolgt:

- Unterwerfen der gesammelten Bahn von Mineralfasern einem Entwirrungsprozess,
- Suspendieren der Mineralfasern in einem Primärluftstrom,
- Mischen des Bindemittels mit den Mineralfasern vor, während oder nach dem Entwirrungsprozess, um eine Mischung aus Mineralfasern und Bindemittel zu bilden.

14. Verfahren zur Herstellung eines Mineralwollprodukts nach irgendeinem der Ansprüche 11-13, wobei der Härtungsprozess ein Trocknungsverfahren umfasst, insbesondere durch Blasen von Luft oder Gas über das Mineralwollprodukt oder durch Erhöhen der Temperatur.

**EP 3 455 184 B1**

**Revendications**

1. Produit en laine minérale comprenant des fibres minérales collées par un liant résultant du durcissement d'une composition de liant sans formaldéhyde pour fibres minérales comprenant :

   - au moins un composé contenant du phénol,
   - au moins une protéine,

   dans lequel la teneur en le composé contenant du phénol est de 1 à 70 % en poids, telle que 2 à 60 % en poids, telle que 3 à 50 % en poids, telle que 4 à 40 % en poids, telle que 5 à 35 % en poids, sur la base des protéines sèches,
   dans lequel l'au moins une protéine est choisie dans le groupe constitué par les protéines d'origine animale, y compris le collagène, la gélatine, la gélatine hydrolysée, et les protéines du lait (caséine, lactosérum), les œufs, les protéines polyphénoliques telles que la protéine de pied de moule.

2. Produit en laine minérale selon la revendication 1, dans lequel l'au moins un composé contenant du phénol comprend un composé contenant du phénol tel que les phénolates simples, tels que les acides hydroxybenzoïques, tels que les aldéhydes hydroxybenzoïques, tels que les hydroxyacétophénones, tels que les acides hydroxyphénylacétiques, tels que les acides cinnamiques, tels que les esters d'acide cinnamique, tels que les cinnamylaldéhydes, tels que les alcools cinnamyliques, tels que les coumarines, tels que les isocoumarines, tels que les chromones, tels que les flavonoïdes, tels que les chalcones, tels que les dihydrochalcones, tels que les aurones, tels que les flavanones, tels que les flavanolols, tels que les flavanes, tels que les leucoanthocyanidines, tels que les flavane-3-ols, tels que les flavones, tels que les anthocyanidines, tels que les désoxyanthocyanidines, tels que les anthocyanines, tels que les biflavonyles, tels que les benzophénones, tels que les xanthones, tels que les stilbènes, tels que les bêtacyanines, tels que les polyphénols et/ou polyhydroxyphénols, tels que les lignanes, néolignanes (dimères ou oligomères issus du couplage de monolignols tels que l'alcool *p*-coumarylique, l'alcool coniférylique et l'alcool sinapylique), tels que les lignines (synthétisées principalement à partir des précurseurs de monolignol alcool *p*-coumarylique, alcool coniférylique et alcool sinapylique), tels que les tanins, tels que les tanins condensés (proanthocyanidines), tels que les tanins hydrolysables, tels que les gallotanins, tels que les ellagitanins, tels que les tanins complexes, tels que l'acide tannique, tels que les phlobabènes.

3. Produit en laine minérale selon la revendication 2, dans lequel le tanin est choisi parmi un ou plusieurs composants à partir du groupe constitué par l'acide tannique, les tanins condensés (proanthocyanidines), les tanins hydrolysables, les gallotanins, les ellagitanins, les tannins complexes, et/ou les tanins issus d'un ou plusieurs parmi le chêne, le châtaigner, le sumac vinaigrier, et le tellime.

4. Produit en laine minérale selon l'une quelconque des revendications précédentes, dans lequel le composé contenant du phénol comprend une ou plusieurs molécules synthétiques ou semi-synthétiques qui contiennent des phénols, polyphénols, quinones, telles que les protéines, peptides, peptoïdes ou arylopeptoïdes modifiés avec des chaînes latérales contenant du phénol, tels que les dendrimères décorés avec des chaînes latérales contenant du phénol.

5. Produit en laine minérale selon la revendication 4, dans lequel le collagène ou la gélatine provient d'une ou plusieurs sources à partir du groupe constitué par les mammifères, les oiseaux, comme les vaches, cochons, chevaux, volailles, et/ou à partir d'écaillés, de peau de poisson.

6. Produit en laine minérale selon l'une quelconque des revendications précédentes, dans lequel le rapport en masse de (lysine + cystéine) dans la protéine au (phénol) dans le composé contenant du phénol est de 1:5,78 à 1:0,08, tel que 1:2,89 à 1:0,09, tel que 1:1,93 à 1:0,12, tel que 1:1,45 à 1:0,15, tel que 1:1,16 à 1:0,17.

7. Produit en laine minérale selon l'une quelconque des revendications précédentes, dans lequel la composition de liant comprend en outre un additif choisi dans le groupe comprenant un oxydant, tel que la tyrosinase, un ion métallique, tel qu'un ion fer, un agent d'ajustement du pH, de préférence sous forme d'une base, telle qu'une base organique, telle qu'une amine ou ses sels, les bases inorganiques, telles qu'un hydroxyde métallique, tel que KOH ou NaOH, l'ammoniac ou ses sels.

8. Produit en laine minérale selon l'une quelconque des revendications précédentes, dans lequel la composition de liant a un pH supérieur à 7, tel que supérieur à 8, tel que supérieur à 9.

**9.** Produit en laine minérale selon la revendication 8, dans lequel la masse volumique du produit en laine minérale est située dans la plage de 10 à 1 200 kg/m$^3$, telle que 30 à 800 kg/m$^3$, telle que 40 à 600 kg/m$^3$, telle que 50 à 250 kg/m$^3$, telle que 60 à 200 kg/m$^3$.

**10.** Produit en laine minérale selon l'une quelconque des revendications 8 et 9, dans lequel la perte au feu (LOI) est située dans la plage de 0,1 à 25,0 %, telle que 0,3 à 18,0 %, telle que 0,5 à 12,0 %, telle que 0,7 à 8,0 % en poids.

**11.** Procédé de production d'un produit en laine minérale selon l'une quelconque des revendications 1 à 7, qui comprend les étapes consistant à mettre des fibres minérales en contact avec la composition de liant, et à durcir le liant, où le durcissement est effectué à une température de 5 à 95°C, telle que 10 à 60°C, telle que 20 à 40°C.

**12.** Procédé de production d'un produit en laine minérale selon la revendication 11, où le procédé comprend les étapes consistant à :

- préparer une masse fondue de matières premières,
- défibrer la masse fondue au moyen d'un dispositif de formation de fibres pour former des fibres minérales,
- obtenir les fibres minérales sous la forme d'une toile collectée,
- mélanger la composition de liant avec les fibres minérales avant, pendant ou après l'obtention de la toile collectée pour former un mélange de fibres minérales et de composition de liant,
- durcir le mélange de fibres minérales et de composition de liant.

**13.** Procédé de production d'un produit en laine minérale selon la revendication 12, dans lequel le mélange du liant avec les fibres minérales est effectué après l'obtention de la toile collectée dans les étapes suivantes consistant à :

- soumettre la toile collectée de fibres minérales à un traitement de démêlage,
- mettre les fibres minérales en suspension dans un courant d'air primaire,
- mélanger le liant avec les fibres minérales avant, pendant ou après le traitement de démêlage pour former un mélange de fibres minérales et de liant.

**14.** Procédé de production d'un produit en laine minérale selon l'une quelconque des revendications 11 à 13, dans lequel le traitement de durcissement comprend un traitement de séchage, en particulier par soufflage d'air ou de gaz sur le produit en laine minérale ou par augmentation de la température.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 583086 A
- EP 990727 A
- EP 1741726 A
- US 5318990 A
- US 20070173588 A
- WO 9936368 A
- WO 0105725 A
- WO 0196460 A
- WO 0206178 A
- WO 2004007615 A
- WO 2006061249 A
- WO 2008023032 A
- WO 2010132641 A1
- DE 4130077 A1
- CN 101302410 A
- EP 2738232 A1
- WO 9719141 A
- GB 926749 A
- US 3824086 A
- WO 8303092 A
- EP 10190521 A

**Non-patent literature cited in the description**

- **C. PEÑA ; K. DE LA CABA ; A. ECEIZA ; R. RUSECKAITE ; I. MONDRAGON.** *Biores. Technol.,* 2010, vol. 101, 6836-6842
- **W. VERMERRIS ; R. NICHOLSON.** Phenolic Compound Biochemistry. Springer, 2008
- **J. J. WILKER.** *Nature Chem. Biol.,* 2011, vol. 7, 579-580